# EUROPEAN PATENT APPLICATION

(11) **EP 4 420 656 A1**
(43) Date of publication of application: **28.08.2024**
(21) Application number: 22883593.0
(22) Date of filing: 19.10.2022
(51) Int. Cl.: A61K 9/16, A61K 31/635, A61K 47/02, A61K 47/32, A61K 47/36, A61K 47/38, A61K 47/42, A61P 29/00

(54) **PHARMACEUTICAL COMPOSITION AND METHOD FOR PRODUCING SAME**

(30) Priority: 20.10.2021 JP 2021171923
(71) Applicant: Sunsho Pharmaceutical Co., Ltd., Fuji-shi, Shizuoka 419-0201 (JP)
(72) Inventor: OZEKI, Toshinori, 4180013 Shizuoka (JP); OOKAWARA, Masaki, 4180013 Shizuoka (JP); SEI, Shunsuke, 4180013 Shizuoka (JP); KURONO, Masahiro, 4180013 Shizuoka (JP); SUGIURA, Mizue, 4180013 Shizuoka (JP)
(74) Representative: Bird & Bird LLP
(86) International application number: PCT/JP2022/038888
(87) International publication number: WO 2023/068289

(57) **Abstract**

Provided is a pharmaceutical composition or the like having enteric properties in a novel form.

A pharmaceutical composition including a core particle containing a medicinal ingredient and a dietary fiber, and a coating particle layer coating the core particle.

## Description

### Technical Field

The present invention relates to a pharmaceutical composition and a method for producing the same.

### Background Art

Conventionally, a technique called drug delivery system (DDS) has been used to enhance a therapeutic effect of a drug and/or to avoid a side effect of the drug. DDS is mainly classified into release control, absorption improvement, and targeting. Among them, as DDS for release control, an enteric preparation in which a drug is not released in the stomach but is released in the intestine is widely known. The enteric preparation can prevent deactivation or decomposition of a medicinal ingredient due to gastric acid or an intragastric digestive enzyme, and occurrence of an intragastric side effect due to the medicinal ingredient.

As such an enteric preparation, for example, Patent Literature 1 describes an invention related to a solid preparation containing an active substance containing esomeprazole or a pharmaceutically acceptable salt thereof and granules having an enteric layer coated with the active substance, in which the enteric layer contains an enteric polymer and macrogol.

Patent Literature 1 describes that, in order to formulate esomeprazole, which is extremely unstable to an acid, a solid preparation having an enteric layer is formed, and macrogol is used as a plasticizer of the enteric layer, whereby an effect of suppressing an increase in analog substances of an active ingredient is obtained.

### Citation List

### Patent Literature

Patent Literature 1: JP 2021-46372 A

### Summary of Invention

### Technical Problem

Under such circumstances, a new enteric preparation is required.

### Solution to Problem

The present invention includes, for example, the following aspects.

[1] A pharmaceutical composition including:
   a core particle containing a medicinal ingredient and a dietary fiber; and
   a coating particle layer coating the core particle.
[2] The pharmaceutical composition according to [1], wherein the dietary fiber contains at least one selected from the group consisting of chitosan, chitin, cellulose, hemicellulose, lignin, and pullulan.
[3] The pharmaceutical composition according to [1] or [2], wherein the core particle further contains a thickener.
[4] The pharmaceutical composition according to [3], wherein the thickener contains at least one selected from the group consisting of inulin, pectin, a pectin processed product, guar gum, a guar gum decomposition product, psyllium seed gum, sterculia urens gum, tragacanth gum, gum arabic, indigestible starch, indigestible dextrin, isomaltodextrin, polydextrose, methyl cellulose, soybean polysaccharides, β-glucan, glucomannan, galactomannan, chondroitin sulfate, hyaluronic acid, levan, lignin, alginic acid and a salt thereof, agarose, gelatin, carrageenan, agar, hydroxypropylmethyl cellulose, hydroxypropylcellulose, an aminoalkyl methacrylate copolymer, and an ammonioalkyl methacrylate copolymer.
[5] The pharmaceutical composition according to any one of [1] to [4], wherein a content of the dietary fiber is 1.0 mass% or more with respect to the total mass of the pharmaceutical composition.
[6] The pharmaceutical composition according to any one of [1] to [5], wherein the coating particle layer contains a divalent or higher valent metal salt.
[7] The pharmaceutical composition according to any one of [1] to [6], which is enteric.
[8] The pharmaceutical composition according to any one of [1] to [7], wherein the dietary fiber is crosslinked.
[9] The pharmaceutical composition according to [8], which is enteric in the large intestine.
[10] The pharmaceutical composition according to any one of [1] to [9], wherein the medicinal ingredient contains at least one selected from the group consisting of mesalazine, salazosulfapyridine, prednisolone, azathioprine, tacrolimus, budesonide, 5-aminosalicylic acid, ustekinumab, sulfasalazine, an anti-TNF antibody, betamethasone, betamethasone sodium phosphate, betamethasone phosphate, azathioprine, 6-mercaptopurine, cyclosporine, and natalizumab.
[11] A method for producing the pharmaceutical composition according to any one of [1] to [10], the method including a granulation step of intermittently discharging a predetermined amount of an aqueous solution for granulation containing a medicinal ingredient, a dietary fiber, and water; charging droplets of the aqueous solution for granulation into a fluidized bed including a coating agent; and causing the coating agent to adhere to a surface of the droplets to allow moisture to be absorbed by the coating agent.
[12] The method according to [11], further including a crosslinking step of crosslinking the dietary fiber with a crosslinking agent.

### Advantageous Effects of Invention

According to the present invention, there is provided a pharmaceutical composition or the like having enteric properties in a novel form.

### Brief Description of Drawings

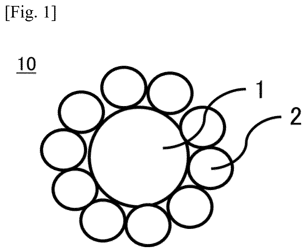

Fig. 1 is a schematic view of a pharmaceutical composition according to an embodiment of the present invention.

### Description of Embodiments

Hereinafter, modes for carrying out the present invention will be described in detail.

### 1. Pharmaceutical Composition

A pharmaceutical composition according to the present invention contains a core particle containing a medicinal ingredient and a dietary fiber, and a coating particle layer coating the core particle.

The pharmaceutical composition of a preferred aspect of the present invention has enteric properties. Thereby, the pharmaceutical composition can enhance a therapeutic effect by the medicinal ingredient and/or can reduce a side effect by the medicinal ingredient. In one embodiment, the pharmaceutical composition exhibits enteric properties in the small intestine. In one embodiment, the pharmaceutical composition exhibits enteric properties in the large intestine. In the present specification, the phrase "in the intestine" means "in the small intestine" and/or "in the large intestine". The term "enteric properties" means enteric properties in the small intestine and/or enteric properties in the large intestine. In the definition of the upper limit value and the lower limit value described in the present specification, a numerical range of the lower limit value to the upper limit value can be defined by appropriately selecting from the respective options and arbitrarily combining them. Various requirements described as preferred aspects described in the present specification can be combined.

Hereinafter, the present invention will be described with reference to the drawings. Note that the drawings may be exaggerated for the sake of explanation, and may differ from actual dimensions.

Fig. 1 is a schematic view of a pharmaceutical composition according to an embodiment of the present invention. A pharmaceutical composition 10 has a core particle 1 containing a medicinal ingredient and a dietary fiber, and a coating particle layer 2 coating the core particle 1. By having the coating particle layer 2 formed by attaching a coating agent to the surface of the core particle 1, the core particle 1 can be granulated into the shape of the pharmaceutical composition 10.

In one embodiment of the present invention, when a solid preparation containing the pharmaceutical composition 10 is taken, at least a part of the coating particle layer 2 is detached or dissolved in the oral cavity, esophagus, or stomach. When a part of the coating particle layer 2 is exfoliated or dissolved, the contact area of the core particle 1 with the gastric juice increases; however, since the dietary fiber constituting the core particle 1 is not digested or hardly digested in the stomach, the shape of the core particle 1 is maintained, or the core particle 1 has an expanded form by absorbing moisture such as saliva or gastric juice. Therefore, the medicinal ingredient in the core particle 1 does not elute or hardly elutes in the stomach. The pharmaceutical composition 10 in which at least a part of the coating particle layer 2 has exfoliated or dissolved in the stomach then migrates into the intestine. The medicinal ingredient of the core particle 1 is eluted by a change in pH in the intestine and/or degradation of dietary fiber by intestinal bacteria. Thereby, the pharmaceutical composition 10 can selectively release the medicinal ingredient in the intestine, that is, exhibits enteric properties.

In one embodiment of the present invention, the medicinal ingredient is contained in the core particle together with the dietary fiber. In this respect, the enteric preparation is different from the conventional enteric preparation in which the medicinal ingredient and the enteric layer are present in different layers. That is, in the pharmaceutical composition of a preferred aspect of the present invention, as compared with the conventional enteric preparation, uneven elution of the medicinal ingredient due to uneven formation of the enteric layer hardly occurs. That is, the pharmaceutical composition according to the present aspect can enable uniform elution of the medicinal ingredient.

In one embodiment of the present invention, the pharmaceutical composition contains a dietary fiber. Therefore, the pharmaceutical composition may have a preventive effect on dyslipidemia, diabetes, colorectal cancer, constipation, and obesity caused by the dietary fiber. When the use of the medicinal ingredient of the pharmaceutical composition is dyslipidemia, diabetes, or colorectal cancer, the efficacy can be synergistically enhanced. When the medicinal ingredient of the pharmaceutical composition has side effects such as constipation and obesity, the side effects can be prevented or reduced.

The average particle size of the pharmaceutical composition is preferably 10 to 1000 µm and more preferably 100 to 500 µm. When the average particle size of the pharmaceutical composition is 10 µm or more, sufficient hardness can be obtained, and the shape can be maintained in a formulation step such as tableting, which is preferable. On the other hand, when the average particle size of the pharmaceutical composition is 1000 µm or less, a tablet or the like to be obtained can have a size that is easy to take, which is preferable. In the present specification, the term "particle size" means the maximum distance among distances between two points on the contour line of an object. The term "average particle size" means an average value of particle sizes of arbitrary 50 objects included in one field of view of a scanning electron microscope (SEM).

Hereinafter, each configuration of the composition of the present invention will be described in detail.

### [Core Particle]

The core particle contains a medicinal ingredient and a dietary fiber. The core particle may further contain a thickener, an enteric polymer, a pH adjusting agent, a solvent, an additive, and the like.

The average particle size of the core particles is preferably 5 to 1000 µm and more preferably 100 to 500 µm. When the average particle size of the core particle is 5 µm or more, the proportion of the core particle can be increased, which is preferable. On the other hand, when the average particle size of the core particle is 1000 µm or less, a tablet or the like to be obtained can have a size that is easy to take, which is preferable.

### (Medicinal Ingredient)

The medicinal ingredient is not particularly limited, but it is preferable that the medicinal ingredient exhibits a therapeutic effect by being absorbed or acted on the small intestine or the large intestine.

The medicinal ingredient contains, for example, at least one selected from the group consisting of mesalazine, salazosulfapyridine, prednisolone, azathioprine, tacrolimus, budesonide, 5-aminosalicylic acid, ustekinumab, sulfasalazine, an anti-TNF antibody, betamethasone, betamethasone sodium phosphate, betamethasone phosphate, azathioprine, 6-mercaptopurine, cyclosporine, and natalizumab.

At this time, the anti-TNF antibody is not particularly limited, and examples thereof include adalimumab, golimumab, infliximab, and certolizumab pegol. The medicinal ingredient may be in the form of a pharmaceutically acceptable salt, solvate, or prodrug thereof, preferably a pharmaceutically acceptable salt or solvate thereof.

Among these medicinal ingredients, it is preferable to contain salazosulfapyridine, prednisolone, tacrolimus, betamethasone, or cyclosporine. The medicinal ingredient may be used singly or in combination of two or more kinds thereof.

In one embodiment, the medicinal ingredient preferably contains at least one selected from the group consisting of a therapeutic agent for dyslipidemia, a therapeutic agent for diabetes, and a therapeutic agent for colorectal cancer.

In one embodiment, the medicinal ingredient preferably has at least one side effect of constipation and obesity.

The content of the medicinal ingredient is preferably 0.01 to 5 mass%, more preferably 0.05 to 3 mass%, and still more preferably 0.1 to 1 mass%, with respect to the total mass of the pharmaceutical composition.

### (Dietary Fiber)

The dietary fiber has a function of preventing or suppressing elution of a medicinal ingredient in the stomach and eluting the medicinal ingredient in the intestine. Whether the medicinal ingredient is eluted in the small intestine (whether the pharmaceutical composition is enteric in the small intestine) or the large intestine (whether the pharmaceutical composition is enteric in the large intestine) can be controlled, for example, by appropriately changing the type, crosslinking degree, and content of the dietary fiber.

The dietary fiber preferably contains at least one selected from the group consisting of chitosan, chitin, cellulose, hemicellulose, lignin, and pullulan, more preferably contains chitosan and/or pullulan, and still more preferably contains chitosan. These dietary fibers may be used singly or in combination of two or more kinds thereof.

The dietary fiber is preferably crosslinked. When the dietary fiber contained in the core particle of the composition of a preferred aspect of the present invention is crosslinked, for example, the moisture absorption degree of the dietary fiber can be lowered. As a result, the core particle is less likely to expand in the stomach, so that the medicinal ingredient is less likely to be eluted in the stomach. Since the cross-linked dietary fiber can be degraded under the influence of pH in the large intestine and/or by intestinal bacteria in the large intestine, when the core particle contains the cross-linked dietary fiber, the resulting pharmaceutical composition can exhibit enteric properties in the large intestine. In the present specification, the presence or absence of crosslinking of the dietary fiber can be checked by observation with a scanning electron microscope (SEM).

The crosslinking degree of the dietary fiber may be, for example, 0.001% or more, 0.01% or more, 0.1% or more, or 1% or more. The crosslinking degree of the dietary fiber may be, for example, 80% or less, 70% or less, 60% or less, 50% or less. In the present specification, the "crosslinking degree of the dietary fiber" can be measured by a swelling test. In the present specification, the upper limit value and the lower limit value can be appropriately combined, and a numerical range indicated by the upper limit value and the lower limit value is also disclosed.

The content of the dietary fiber is preferably 0.5 mass% or more, more preferably 1.0 mass% or more, and still more preferably 1.5 mass% or more, with respect to the total mass of the pharmaceutical composition, and is particularly preferably 2.5 mass% or more from the viewpoint of further enhancing enteric properties. When the content of the dietary fiber is 0.5 mass% or more, dissolution of the medicinal ingredient in the stomach can be suppressed or prevented, which is preferable. The upper limit value of the content of the dietary fiber is not particularly limited, and is preferably 20 mass% or less, more preferably 10 mass% or less, particularly preferably 5 mass% or less, and most preferably 4 mass% or less, with respect to the total mass of the pharmaceutical composition. In one preferred embodiment, when the pharmaceutical composition is enteric in the small intestine, the content of the dietary fiber is preferably 0.5 to 20 mass%, more preferably 0.5 to 10 mass%, still more preferably 0.5 to 5 mass%, particularly preferably 1 to 5 mass%, and most preferably 1 to 3 mass%, with respect to the total mass of the pharmaceutical composition. In another preferred embodiment, when the pharmaceutical composition is enteric in the large intestine, the content of the dietary fiber is preferably 0.5 to 20 mass%, more preferably 1.2 to 20 mass%, still more preferably 1.5 to 10 mass%, particularly preferably 2 to 10 mass%, and most preferably 3 to 5 mass%, with respect to the total mass of the pharmaceutical composition.

### (Thickener)

The core particle may further contain a thickener. The thickener has a function of adjusting the shape of the core particle, a function of adjusting the physical properties (hardness and the like) of the pharmaceutical composition, and the like.

The thickener preferably contains at least one selected from the group consisting of inulin, pectin, a pectin processed product, guar gum, a guar gum decomposition product, psyllium seed gum, sterculia urens gum, tragacanth gum, gum arabic, indigestible starch, indigestible dextrin, isomaltodextrin, polydextrose, methyl cellulose, soybean polysaccharides, β-glucan, glucomannan, galactomannan, chondroitin sulfate, hyaluronic acid, levan, lignin, alginic acid and a salt thereof, agarose, gelatin, carrageenan, agar, hydroxypropylmethyl cellulose, hydroxypropylcellulose, an aminoalkyl methacrylate copolymer, and an ammonioalkyl methacrylate copolymer, more preferably contains at least one selected from the group consisting of alginic acid, sodium alginate, gelatin, and guar gum, and still more preferably contains sodium alginate. The thickener may be used singly or in combination of two or more kinds thereof.

The content of the thickener is preferably 1 to 20 mass%, more preferably 1 to 12 mass%, and still more preferably 2 to 10 mass%, with respect to the total mass of the pharmaceutical composition. When the content of the thickener is 1 mass% or more, the shape can be easily maintained until granulation is performed after discharge, which is preferable. On the other hand, when the content of the thickener is 20 mass% or less, the aqueous solution for granulation can have a viscosity suitable for discharge, which is preferable. In one preferred embodiment, when the pharmaceutical composition is enteric in the small intestine, the content of the thickener is preferably 0.5 to 20 mass%, more preferably 0.5 to 10 mass%, still more preferably 0.5 to 5 mass%, particularly preferably 0.5 to 4 mass%, and most preferably 1 to 4 mass%, with respect to the total mass of the pharmaceutical composition. In another preferred embodiment, when the pharmaceutical composition is enteric in the large intestine, the content of the thickener is preferably 0.5 to 20 mass%, more preferably 3 to 20 mass%, still more preferably 4 to 20 mass%, particularly preferably 4 to 12 mass%, and most preferably 4 to 8 mass%, with respect to the total mass of the pharmaceutical composition.

A ratio of the content (mass%) of the thickener to the content (mass%) of the dietary fiber (thickener/dietary fiber) is preferably 0.1 to 15, more preferably 0.3 to 10, still more preferably 0.3 to 6, particularly preferably 0.3 to 3, very preferably 0.6 to 2, and most preferably 0.7 to 1.5.

### (Enteric Polymer)

The core particle may further contain an enteric polymer. The enteric polymer means a polymer other than the dietary fiber, which does not dissolve in the stomach but dissolves in the intestine. The enteric polymer has, for example, a function of adjusting enteric properties of the pharmaceutical composition by being contained together with the dietary fiber.

The enteric polymer is not particularly limited, and examples thereof include shellac, cellulose acetate phthalate (CAP), hydroxypropyl methyl cellulose phthalate (HPMCP), carboxymethyl ethyl cellulose (CMEC), polyvinyl alcohol phthalate, and a methacrylic acid copolymer. These enteric polymers may be used singly or in combination of two or more kinds thereof.

The content of the enteric polymer is preferably 20 mass% or less, more preferably 10 mass% or less, still more preferably 5 mass% or less, and particularly preferably 0.1 to 5 mass%, with respect to the total mass of the pharmaceutical composition.

### (pH Adjusting Agent)

The core particle may further contain a pH adjusting agent. The pH adjusting agent has a function of improving the stability of the active ingredient by controlling the pH in the core particle.

The pH adjusting agent is not particularly limited, and examples thereof include lactic acid, acetic acid, acetates (such as sodium acetate), citric acid, citrates (such as sodium citrate), phosphoric acid, phosphates (such as sodium phosphate), diisopropanolamine, triisopropanolamine, triethanolamine, potassium hydroxide, and sodium hydroxide. These pH adjusting agents may be used singly or in combination of two or more kinds thereof.

### (Solvent)

The core particle may contain a solvent. The solvent has a function of adjusting dispersibility of the active ingredient, hardness of the pharmaceutical composition, and the like.

Examples of the solvent include water and an organic solvent.

The organic solvent is not particularly limited, and examples thereof include propylene glycol monocaprylate, propylene glycol dicaprylate, propylene glycol dicaprate, propylene glycol monolaurate, propylene glycol monooleate, benzyl benzoate, octyldecyl triglyceride, oleic acid, triethyl citrate, dimethylpolysiloxane, cinnamaldehyde, medium-chain monodiglyceride, medium-chain fatty acid triglyceride, triacetin, piperonyl butoxide, diethyl phthalate, dibutyl phthalate, butyl phthalyl butyl glycolate, octyldodecyl myristate, ethyl butyrate, ethanol, and isopropanol.

Among them, the solvent preferably contains water. The solvent may be used singly or in combination of two or more kinds thereof.

The content of the solvent is preferably 1 to 40 mass%, more preferably 3 to 20 mass%, and still more preferably 5 to 15 mass%, with respect to the total mass of the pharmaceutical composition.

### (Additive)

The core particle may contain an additive.

The additive is not particularly limited, and examples thereof include a colorant, a masking agent, a sweetener, an antioxidant, a preservative, a lubricant, a flavor, and a crosslinking agent. These additives may be used singly or in combination of two or more kinds thereof.

### [Coating Particle Layer]

The coating particle layer of the composition of the present invention coats the core particle. The coating particle layer contains a coating agent. The coating agent adheres to the surface of the core particle to form a layered form, thereby forming a coating particle layer. The coating particle layer of the present invention is layered, but includes a form completely coating the core particle and a form partially coating the core particle. In the form partially coating the core particle, a part of the core particle not coated with the coating particle layer is exposed.

The coating agent is not particularly limited, but is usually water-insoluble particles. Specific examples of the coating agent include a divalent or higher valent metal salt, an inorganic substance, polysaccharides or a derivative thereof, and an organic polymer.

Examples of the divalent or higher valent metal salt include calcium chloride, calcium carbonate, calcium oxide, calcium sulfate, calcium hydrogen phosphate, calcium dihydrogen phosphate, calcium stearate, magnesium chloride, magnesium carbonate, magnesium oxide, magnesium silicate, aluminum chloride, aluminum hydroxide, aluminum phosphate, aluminum sulfate, aluminum silicate, potassium alum, iron chloride alum, ammonium alum, ferric sulfate, iron citrate, zinc oxide, zinc sulfate, and hydrates thereof.

Examples of the inorganic substance include anhydrous silicic acid, silicon dioxide, titanium oxide, kaolin, diatomaceous earth, bentonite, zeolite, and talc.

Examples of the polysaccharides or a derivative thereof include corn starch, rice starch, wheat starch, potato starch, agar powder, crystalline cellulose, ethyl cellulose, croscarmellose sodium, sodium carboxymethyl starch with a low degree of substitution, and sodium starch glycolate.

Examples of the organic polymer include crospovidone, aminoalkyl methacrylate copolymer E, methacrylic acid copolymer L, dry methacrylic acid copolymer LD, methacrylic acid copolymer S, and an ammonioalkyl methacrylate copolymer.

Among these, the coating agent preferably contains a divalent or higher valent metal salt, more preferably contains at least one selected from the group consisting of calcium hydrogen phosphate, calcium dihydrogen phosphate, calcium oxide, magnesium oxide, and aluminum silicate, and still more preferably contains calcium hydrogen phosphate and/or calcium dihydrogen phosphate. The coating agent may be used singly or in combination of two or more kinds thereof.

The average particle size of the coating agent is preferably 1 to 100 µm, more preferably 2 to 50 µm, and still more preferably 3 to 30 µm. When the average particle size of the coating agent is 1 µm or more, the fluidity of the coating agent can be enhanced, which is preferable. On the other hand, when the average particle size of the coating agent is 100 µm or less, the bonding strength with the core particle can be increased due to a small surface area, which is preferable.

The content of the coating agent is preferably less than 95 mass%, more preferably less than 90 mass%, and still more preferably less than 85 mass%, with respect to the total mass of the pharmaceutical composition. When the content of the coating agent is less than 95 mass%, the shape stability of the pharmaceutical composition can be enhanced, which is preferable. The lower limit value of the content of the coating agent is not particularly limited, but is preferably 1 mass% or more, more preferably 20 mass% or more, still more preferably 40 mass% or more, particularly preferably 60 mass% or more, and particularly preferably 70 mass% or more, with respect to the total mass of the pharmaceutical composition. In one preferred embodiment, the content of the coating agent is preferably 20 mass% or more and less than 95 mass%, 60 mass% or more and less than 90 mass%, still more preferably 70 mass% or more and less than 90 mass%, particularly preferably 70 mass% or more and less than 85 mass%, with respect to the total mass of the pharmaceutical composition.

### 2. Method for Producing Pharmaceutical Composition

According to one aspect of the present invention, a method for producing a pharmaceutical composition is provided. The production method of the present invention includes a granulation step of intermittently discharging a predetermined amount of an aqueous solution for granulation containing a medicinal ingredient, a dietary fiber, and water, charging droplets of the aqueous solution for granulation into a fluidized bed including a coating agent, and causing the coating agent to adhere to a surface of the droplets to allow moisture to be absorbed by the coating agent. The production method may further include a crosslinking step of crosslinking the dietary fiber with a crosslinking agent. According to the production method, a pharmaceutical composition having a form in which a medicinal ingredient is contained in a core particle together with a dietary fiber can be produced.

### [Granulation Step]

The granulation step can use, for example, the granulation method disclosed in JP 2019-073459 A, and specifically is a step of intermittently discharging a predetermined amount of an aqueous solution for granulation containing a medicinal ingredient, a dietary fiber, and water, charging droplets of the aqueous solution for granulation into a fluidized bed including a coating agent, and causing the coating agent to adhere to a surface of the droplets to allow moisture to be absorbed by the coating agent.

### (Aqueous Solution for Granulation)

The aqueous solution for granulation contains a medicinal ingredient, a dietary fiber, and water. The aqueous solution for granulation may further contain a thickener, an enteric polymer, a pH adjusting agent, a crosslinking agent, a solvent, an additive, and the like. The aqueous solution for granulation may be contained in the core particle of the pharmaceutical composition.

The components such as a medicinal ingredient, a dietary fiber, a thickener, an enteric polymer, a pH adjusting agent, a solvent, and an additive are as described above.
(medicinal ingredient, dietary fiber, thickener, enteric polymer, pH adjusting agent, solvent, and additive)

The content of each component such as a medicinal ingredient, a dietary fiber, a thickener, an enteric polymer, a pH adjusting agent, a solvent, and an additive is preferably adjusted to an amount such that the content of the pharmaceutical composition in the core particle falls within the above range.

In particular, the content of water is preferably 60 to 98 mass%, more preferably 70 to 95 mass%, and still more preferably 80 to 90 mass%, with respect to the total mass of the aqueous solution for granulation. When the content of water is in the above range, the form of the pharmaceutical composition can be suitably formed when the droplets of the aqueous solution for granulation are charged into a fluidized bed including a coating agent described below, which is preferable.

### (Crosslinking Agent)

The aqueous solution for granulation may contain a crosslinking agent. When the crosslinking agent is contained in the aqueous solution for granulation, the crosslinking step can be performed immediately after the granulation step. In some cases, at least a part of dietary fiber may cause a crosslinking reaction with the crosslinking agent at any time point in the granulation step.

The crosslinking agent is not particularly limited, and examples thereof include dicarboxylic acid compounds such as fumaric acid, maleic acid, citric acid, and succinic acid; α,β-unsaturated aldehyde compounds such as acrolein, methacrolein, crotonaldehyde, and cinnamaldehyde; dialdehyde compounds such as glutaraldehyde, malondialdehyde, and glyoxal; acrylamide; and dimethacrylic acid. Among these, the crosslinking agent preferably contains an α,β-unsaturated aldehyde compound and a dialdehyde compound, more preferably contains crotonaldehyde, cinnamaldehyde, glutaraldehyde, malondialdehyde, and glyoxal, still more preferably contains cinnamaldehyde and glutaraldehyde, and particularly preferably contains glutaraldehyde. These crosslinking agents may be used singly or in combination of two or more kinds thereof.

The content of the crosslinking agent is preferably 1 to 30 mass%, more preferably 2 to 20 mass%, and still more preferably 3 to 15 mass%, with respect to the total mass of the aqueous solution for granulation.

The viscosity of the aqueous solution for granulation is not particularly limited, and is preferably 0.5 to 20000 mPa and more preferably 1 to 10000 mPa. When the viscosity of the aqueous solution for granulation is 0.5 mPa or more, the shape is easy to maintain when the droplets of the discharged aqueous solution for granulation are charged into a fluidized bed, which is preferable. When the viscosity of the aqueous solution for granulation is 20000 mPa or less, productivity is excellent, which is preferable.

### (Discharge of Aqueous Solution for Granulation)

The aqueous solution for granulation is intermittently discharged by a predetermined amount at a time. As this time, the discharged aqueous solution for granulation has a droplet shape. The discharge of the aqueous solution for granulation can be performed by, for example, a liquid metering dispenser including a discharge nozzle, or the like.

The discharge amount per one time of the aqueous solution for granulation can be usually controlled by the nozzle diameter and the discharge pressure of the discharge nozzle of the liquid metering dispenser. The average particle size of the core particle can be controlled by adjusting the discharge amount of the aqueous solution for granulation.

The nozzle diameter of the discharge nozzle is not particularly limited, and is preferably 0.1 to 1.0 mm and more preferably 0.2 to 0.5 mm. When the nozzle diameter of the discharge nozzle is 0.1 mm or more, it is preferable from the viewpoint of being able to discharge even a highly viscous aqueous solution for granulation, excellent productivity, and the like. On the other hand, when the nozzle diameter of the discharge nozzle is 1.0 mm or less, the shape of the droplet can be maintained when the liquid droplet comes into contact with the fluidized bed, which is preferable.

The discharge pressure is not particularly limited, and is preferably 0.1 to 5.0 MPa and more preferably 0.1 to 2.0 MPa. When the discharge pressure is 0.1 MPa or more, it is preferable from the viewpoint of being able to discharge even a highly viscous aqueous solution for granulation, excellent productivity, and the like. On the other hand, when the discharge pressure is 5.0 MPa or less, the shape of the droplet can be maintained when the liquid droplet comes into contact with the fluidized bed, which is preferable

The time (intermittent time) of the discharge interval of the aqueous solution for granulation is usually controlled by an intermittent discharge rate of a liquid metering dispenser. The intermittent discharge rate is not particularly limited, and is preferably 500 to 50000 rpm and more preferably 1000 to 20000 rpm. When the intermittent discharge rate is 500 rpm or more, productivity can be enhanced, which is preferable. On the other hand, when the intermittent discharge rate is 50000 rpm or less, heat generation of the device due to friction can be suppressed, which is preferable. In the present specification, the "intermittent discharge rate" means the number of discharges per minute.

### (Charging into Fluidized Bed)

The droplets of the aqueous solution for granulation are charged into a fluidized bed. A coating agent is stored in the fluidized bed, and the coating agent adheres to the surface of the droplet when the droplet comes into contact with the coating agent of the fluidized bed. Subsequently, the attached coating agent absorbs moisture in the droplets, so that fluidity of the droplets is reduced to form a core particle. The coating agent adheres to the core particle has a layered configuration to form a coating particle layer. As a result, a pharmaceutical composition can be produced. The coating agent is as described above.

Examples of the fluidized bed include a bed in which a coating agent is stored in a pan granulator and rolled. When the rotating pan (dish) gives the coating agent for a rolling operation, the coating agent can have a layered configuration when the droplets come into contact with the coating agent. Collision with a subsequent droplet can be prevented or suppressed.

The vibration applied to the coating agent in the fluidized bed is not particularly limited, and is preferably 10 to 500 rpm and more preferably 20 to 200 rpm. When the vibration is 10 rpm or more, collision with a subsequent droplet can be prevented or suppressed, which is preferable. On the other hand, when the vibration is 500 rpm or less, the coated particles can be suppressed from being biased by a centrifugal force, which is preferable.

### [Crosslinking Step]

In one embodiment, the method for producing a pharmaceutical composition may further include a crosslinking step. By having the crosslinking step, the dietary fiber is crosslinked, and a pharmaceutical composition exhibiting enteric properties, preferably enteric properties in the large intestine, can be produced.

The crosslinking method is not particularly limited, and examples thereof include a method in which the pharmaceutical composition obtained in the granulation step is brought into contact with a crosslinking agent, and a method in which the dietary fiber contained in the pharmaceutical composition obtained in the granulation step and a crosslinking agent are reacted. As the crosslinking agent, those described above can be used.

The method of bringing the pharmaceutical composition into contact with a crosslinking agent is not particularly limited, and examples thereof include a method in which the pharmaceutical composition is immersed in a solution containing a crosslinking agent, and a method in which a solution containing a crosslinking agent is sprayed onto the pharmaceutical composition. For example, when the dietary fiber contains chitosan, chitosan can be crosslinked by immersing the pharmaceutical composition in an aqueous solution of glutaraldehyde. More specifically, an amino group of chitosan can be crosslinked by forming an imine with a carbonyl group of glutaraldehyde. Although the pharmaceutical composition has a coating particle layer, since voids may be present in the coating particle layer, when the pharmaceutical composition is immersed in an aqueous solution of glutaraldehyde, chitosan in the core particle can react with glutaraldehyde.

When the aqueous solution for granulation contains a crosslinking agent, the pharmaceutical composition obtained in the granulation step contains a dietary fiber and a crosslinking agent. In this case, the dietary fiber can be crosslinked by reacting the dietary fiber with the crosslinking agent in the crosslinking step.

The crosslinking temperature is not particularly limited, and is preferably 10 to 80°C and more preferably 15 to 75°C.

The crosslinking time is not particularly limited, and is preferably 6 to 72 hours and more preferably 12 to 48 hours.

### [Modification]

The method for producing a pharmaceutical composition described above can be appropriately changed according to the type of the material to be used, desired physical properties, and the like. The modification of the method for producing a pharmaceutical composition described above includes a pre-granulation crosslinking step of crosslinking a dietary fiber in an aqueous solution for granulation containing a medicinal ingredient, a dietary fiber, a crosslinking agent, and water, and a granulation step of charging droplets of the aqueous solution for granulation containing the crosslinked dietary fiber into a fluidized bed including a coating agent, and causing the coating agent to adhere to a surface of the droplets to allow moisture to be absorbed by the coating agent. At this time, a post-granulation crosslinking step of crosslinking the dietary fiber may be further included. That is, in the modification, the dietary fiber in the aqueous solution for granulation is crosslinked before granulation.

### (Pre-Granulation Crosslinking Step)

The pre-granulation crosslinking step is a step of crosslinking a dietary fiber of an aqueous solution for granulation containing a medicinal ingredient, a dietary fiber, a crosslinking agent, and water. The crosslinking temperature and the crosslinking time are as described above.

### (Granulation Step)

The granulation step is a step of charging droplets of the aqueous solution for granulation containing the crosslinked dietary fiber into a fluidized bed including a coating agent, and causing the coating agent to adhere to a surface of the droplets to allow moisture to be absorbed by the coating agent. The granulation step can be performed by the same method as the method described above.

### (Post-Granulation Crosslinking Step)

The modification may further include a post-granulation crosslinking step. The post-granulation crosslinking step can be performed by the same method as the method of the crosslinking step described above. That is, the pharmaceutical composition may be brought into contact with a newly prepared crosslinking agent, or the dietary fiber and the crosslinking agent remaining in the pharmaceutical composition obtained in the granulation step may be further reacted.

### 3. Solid Preparation

According to one aspect of the present invention, a solid preparation containing a pharmaceutical composition is provided. The dosage form of the solid preparation is not particularly limited, and examples thereof include tablets, capsules, powders, granules, and granules. Among these, the solid preparation is preferably a tablet.

Tablets are usually formed by compressing the pharmaceutical composition. At this time, an excipient, a disintegrant, a binder, a lubricant, a stabilizer, a suspending agent, a coating agent, an antioxidant, a dispersant, a fluidizing agent, a flavor, a sweetener, a flavoring agent, a colorant, and the like may be added together with the pharmaceutical composition, and compression molding may be performed.

### Examples

Hereinafter, the present invention will be specifically described with reference to Examples; however, the present invention is not limited thereto.

### [Example 1]

### (Granulation Step)

An aqueous solution for granulation was prepared. First, salazosulfapyridine (SASP) and chitosan as medicinal ingredients were added to a 0.4% acetic acid aqueous solution to obtain a mixed solution. Subsequently, sodium alginate was added to the mixed solution, and a 1 N sodium hydroxide (NaOH) aqueous solution was added thereto to adjust the pH to 6.5, thereby obtaining an aqueous solution for granulation. The composition of the aqueous solution for granulation was as shown in Table 1 below.

**[Table 1]**

| | Content (mass%) |
|---|---|
| SASP* | 0.90 |
| Chitosan | 1.78 |
| Sodium alginate | 7.23 |
| Acetic acid | 0.003 |
| NaOH | 0.03 |
| Water | 90.06 |
| Total | 100 |

| | |
|---|---|
| *SASP: salazosulfapyridine | |

Into a liquid metering dispenser (discharge nozzle diameter: 0.3 mm), 20 g of the prepared aqueous solution for granulation was charged, and the discharge pressure was set to 0.5 MPa and the intermittent discharge rate was set to 10000 rpm.

An open pan granulator (pan diameter: 300mm) was installed 70 cm below the discharge nozzle, and 250 g of anhydrous dibasic calcium phosphate (average particle size: 5.3 µm) was stored in the open pan granulator. The open pan granulator was revolvingly vibrated at 90 rpm to form a fluidized bed in which anhydrous dibasic calcium phosphate was rotationally fluidized.

From the liquid metering dispenser, 13.8 g of the aqueous solution for granulation was intermittently discharged, and droplets of the aqueous solution for granulation were charged into a fluidized bed. A pharmaceutical composition having a core particle derived from an aqueous solution for granulation and a coating particle layer of calcium hydrogen phosphate was produced by attaching anhydrous dibasic calcium phosphate to the surface of a droplet and absorbing moisture of the droplet. The pharmaceutical composition and the anhydrous dibasic calcium phosphate in the open pan granulator were separated by a sieve (mesh size: 106 µm).

### [Example 2]

### (Granulation Step)

A pharmaceutical composition having a core particle and a coating particle layer of calcium hydrogen phosphate was produced by the same method as in Example 1.

### (Crosslinking Step)

Chitosan contained in the core particles of the pharmaceutical composition was crosslinked.

Specifically, the pharmaceutical composition was immersed in a 2% glutaraldehyde aqueous solution for 24 hours. After immersion, the pharmaceutical composition was fractionated and dried with dry air to produce a pharmaceutical composition crosslinked with chitosan.

### [Example 3]

### (Granulation Step)

A pharmaceutical composition having a core particle and a coating particle layer of calcium hydrogen phosphate was produced by the same method as in Example 1, except that an aqueous solution for granulation shown in Table 2 below was used.

**[Table 2]**

| | Content (mass%) |
|---|---|
| SASP* | 0.90 |
| Chitosan | 7.23 |
| Sodium alginate | 4.92 |
| Acetic acid | 0.003 |
| NaOH | 0.03 |
| Water | 86.92 |
| Total | 100 |

| | |
|---|---|
| *SASP: salazosulfapyridine | |

### [Example 4]

### (Granulation Step)

A pharmaceutical composition having a core particle and a coating particle layer of calcium hydrogen phosphate was produced by the same method as in Example 3.

### (Crosslinking Step)

Chitosan contained in the core particle of the pharmaceutical composition was crosslinked by the same method as in Example 2 to produce a pharmaceutical composition crosslinked with chitosan.

### [Example 5]

### (Granulation Step)

A pharmaceutical composition having a core particle and a coating particle layer of calcium hydrogen phosphate was produced by the same method as in Example 1, except that an aqueous solution for granulation shown in Table 3 below was used.

**[Table 3]**

| | Content (mass%) |
|---|---|
| SASP* | 0.90 |
| Chitosan | 7.23 |
| Sodium alginate | 7.23 |
| Acetic acid | 0.003 |
| NaOH | 0.03 |
| Water | 84.60 |
| Total | 100 |

| | |
|---|---|
| *SASP: salazosulfapyridine | |

### [Example 6]

### (Granulation Step)

A pharmaceutical composition having a core particle and a coating particle layer of calcium hydrogen phosphate was produced by the same method as in Example 5.

### (Crosslinking Step)

Chitosan contained in the core particle of the pharmaceutical composition was crosslinked by the same method as in Example 2 to produce a pharmaceutical composition crosslinked with chitosan.

### [Example 7]

### (Granulation Step)

An aqueous solution for granulation was prepared. First, salazosulfapyridine (SASP) and chitosan as medicinal ingredients were added to a 0.4% acetic acid aqueous solution to obtain a mixed solution. Subsequently, sodium alginate and cinnamaldehyde were added to the mixed solution, and a 1 N sodium hydroxide (NaOH) aqueous solution was added thereto to adjust the pH to 6.5, thereby obtaining an aqueous solution for granulation. The composition of the aqueous solution for granulation was as shown in Table 4 below.

**[Table 4]**

| | Content (mass%) |
|---|---|
| SASP* | 0.90 |
| Chitosan | 9.90 |
| Sodium alginate | 8.10 |
| Acetic acid | 0.003 |
| NaOH | 0.03 |
| Cinnamaldehyde | 10.00 |
| Water | 71.07 |
| Total | 100 |

| | |
|---|---|
| *SASP: salazosulfapyridine | |

In the same manner as in Example 1, the prepared aqueous solution for granulation and anhydrous dibasic calcium phosphate were used to produce a pharmaceutical composition having core particles derived from the aqueous solution for granulation and a coating particle layer of calcium hydrogen phosphate.

### (Crosslinking Step)

Chitosan contained in the core particles of the pharmaceutical composition was crosslinked.

Specifically, a pharmaceutical composition crosslinked with chitosan was produced by heating the pharmaceutical composition at 75°C for 6 to 72 hours.

### [Example 8]

### (Granulation Step)

A pharmaceutical composition having a core particle and a coating particle layer of calcium hydrogen phosphate was produced in the same manner as in Example 7, except that an aqueous solution for granulation shown in Table 5 below was used.

**[Table 5]**

| | Content (mass%) |
|---|---|
| SASP* | 0.90 |
| Chitosan | 11.70 |
| Sodium alginate | 5.85 |
| Acetic acid | 0.003 |
| NaOH | 0.03 |
| Cinnamaldehyde | 10.00 |
| Water | 71.52 |
| Total | 100 |

| | |
|---|---|
| *SASP: salazosulfapyridine | |

### (Crosslinking Step)

Chitosan contained in the core particle of the pharmaceutical composition was crosslinked by the same method as in Example 7 to produce a pharmaceutical composition crosslinked with chitosan.

### [Reference Example 1]

Instead of salazosulfapyridine (SASP) as a medicinal ingredient, Red No. 102 as an additive was used. Specifically, a pharmaceutical composition crosslinked with chitosan was produced by the same method as in Example 2, except that the composition of the aqueous solution for granulation was as shown in Table 6 below.

**[Table 6]**

| | Content (mass%) |
|---|---|
| Red No. 102 | 0.80 |
| Chitosan | 2.50 |
| Sodium alginate | 12.5 |
| Acetic acid | 0.003 |
| NaOH | 0.03 |
| Water | 84.17 |
| Total | 100 |

### [Reference Example 2]

Instead of salazosulfapyridine (SASP) as a medicinal ingredient, Red No. 102 as an additive was used. Specifically, a pharmaceutical composition crosslinked with chitosan was produced by the same method as in Example 2, except that the aqueous solution for granulation in Table 7 below was used.

**[Table 7]**

| | Content (mass%) |
|---|---|
| Red No. 102 | 1.30 |
| Chitosan | 1.70 |
| Sodium alginate | 12.5 |
| Acetic acid | 0.003 |
| NaOH | 0.03 |
| Water | 84.54 |
| Total | 100 |

### [Reference Example 3]

Instead of salazosulfapyridine (SASP) as a medicinal ingredient, Red No. 102 as an additive was used. Specifically, a pharmaceutical composition crosslinked with chitosan was produced by the same method as in Example 2, except that the aqueous solution for granulation in Table 8 below was used.

**[Table 8]**

| | Content (mass%) |
|---|---|
| Red No. 102 | 1.3 |
| Chitosan | 1.3 |
| Sodium alginate | 12.6 |
| Acetic acid | 0.003 |
| NaOH | 0.03 |
| Water | 84.77 |
| Total | 100 |

### [Comparative Example 1]

Instead of salazosulfapyridine (SASP) as a medicinal ingredient, Red No. 102 as an additive was used and chitosan was not used. Specifically, a pharmaceutical composition was produced by the same method as in Example 1, except that the aqueous solution for granulation in Table 9 below was used.

**[Table 9]**

| | Content (mass%) |
|---|---|
| Red No. 102 | 0.8 |
| Chitosan | 0 |
| Sodium alginate | 12.5 |
| Acetic acid | 0.003 |
| NaOH | 0.03 |
| Water | 86.67 |
| Total | 100 |

The compositions of the pharmaceutical compositions produced in Examples 1 to 8, Reference Examples 1 to 3, and Comparative Example 1 are shown in Table 10 below.

**[Table 10]**

| | Core particle | | | | | | | | | | | | Coating layer | |
|---|---|---|---|---|---|---|---|---|---|---|---|---|---|---|
| | Medicinal ineredient/additive | | Dietary fiber (chitosan) | | | Thickener | | pH adjusting agent | | | | Water | | |
| | Type | Content (mass%) | Content (mass%) | Presence or absence of crosslinking | Crosslinking agent | Type | Content (mass%) | Type | Content (mass%) | Type | Content (mass%) | Content (mass%) | Type | Content (mass%) |
| Example 1 | SASP | 0.50 | 1.17 | × | - | Sodium alginate | 4.74 | NaOH | 0.02 | Acetic acid | 0.002 | 10 | Calcium hydrogen phosphate | 83.57 |
| Example 2 | SASP | 0.36 | 1.17 | ο | Glutaraldehyde | Sodium alginate | 4.74 | NaOH | 0.02 | Acetic acid | 0.002 | 10 | Calcium hydrogen phosphate | 83.71 |
| Example 3 | SASP | 0.59 | 4.74 | × | - | Sodium alginate | 3.23 | NaOH | 0.02 | Acetic acid | 0.002 | 10 | Calcium hydrogen phosphate | 81.42 |
| Example 4 | SASP | 0.26 | 4.74 | ○ | Glutaraldehyde | Sodium alginate | 3.23 | NaOH | 0.02 | Acetic acid | 0.002 | 10 | Calcium hydrogen phosphate | 81.76 |
| Example 5 | SASP | 0.59 | 4.74 | × | - | Sodium alginate | 4.74 | NaOH | 0.02 | Acetic acid | 0.002 | 10 | Calcium hydrogen phosphate | 79.91 |
| Example 6 | SASP | 0.24 | 4.74 | ○ | Glutaraldehyde | Sodium alginate | 4.74 | NaOH | 0.02 | Acetic acid | 0.002 | 10 | Calcium hydrogen phosphate | 80.26 |
| Example 7 | SASP | 0.43 | 4.74 | ○ | Cinnamaldehyde | Sodium alginate | 3.88 | NaOH | 0.02 | Acetic acid | 0.002 | 10 | Calcium hydrogen phosphate | 80.93 |
| Example 8 | SASP | 0.71 | 5.60 | ○ | Cinnamaldehyde | Sodium alginate | 2.80 | NaOH | 0.02 | Acetic acid | 0.002 | 10 | Calcium hydrogen phosphate | 81.15 |
| Reference Example 1 | Red No. 102 | 0.09 | 2.28 | ○ | Glutaraldehyde | Sodium alginate | 11.40 | NaOH | 0.02 | Acetic acid | 0.002 | 10 | Calcium hydrogen phosphate | 76.21 |
| Reference Example 2 | Red No. 102 | 0.37 | 1.63 | ○ | Glutaraldehyde | Sodium alginate | 12.20 | NaOH | 0.02 | Acetic acid | 0.002 | 10 | Calcium hydrogen phosphate | 75.78 |
| Reference Example 3 | Red No. 102 | 0.44 | 1.45 | ○ | Glutaraldehyde | Sodium alginate | 13.02 | NaOH | 0.02 | Acetic acid | 0.002 | 10 | Calcium hydrogen phosphate | 75.07 |
| Comparative Example 1 | Red No. 102 | 0.08 | 0 | - | | Sodium alginate | 13.94 | NaOH | 0.02 | Acetic acid | 0.002 | 10 | Calcium hydrogen phosphate | 75.96 |

| | | | | | | | | | | | | | | |
|---|---|---|---|---|---|---|---|---|---|---|---|---|---|---|
| *SASP: salazosulfapyridine | | | | | | | | | | | | | | |

### [Evaluation]

For the pharmaceutical compositions produced in Examples 1 to 8, Reference Examples 1 to 3, and Comparative Example 1, gastric solubility, enteric properties in the small intestine, and enteric properties in the large intestine were evaluated.

### (Evaluation of Gastric Solubility)

A first solution for dissolution test (hereinafter, also referred to as "liquid I") was prepared by dissolving 2.0 g of sodium chloride in 7.0 mL of hydrochloric acid and water to make 1000 mL. The pH of the liquid I was 1.2, which was equivalent to the pH in the stomach.

Using an apparatus for the paddle method described in Dissolution Test Method (6.10) in The Japanese Pharmacopoeia 18th edition, 200 mg of the pharmaceutical composition was added to the liquid I stirred at 37°C and 50 rpm. After addition, the liquid I was fractionated after 15 minutes, 30 minutes, 60 minutes, and 120 minutes. The amount of the medicinal ingredient in the liquid I was measured by high-performance liquid chromatography (HPLC), and the dissolution rate (%) (dissolution rate of the liquid I) of salazosulfapyridine or Red No. 102 eluted in the liquid I from the pharmaceutical composition was calculated. The measurement conditions of HPLC are as follows.

### <Measurement Conditions of HPLC of Examples 1 to 8>

Apparatus: Prominence (manufactured by SHIMADZU CORPORATION)
Column: Inertsil ODS (manufactured by GL Sciences Inc.)
Developing solvent: methanol: 10 mM ammonium acetate aqueous solution = 48 : 52
Measurement sample: 1 mL of liquid I and 1 mL of developing solvent were mixed
Quantification method: performed by ultraviolet-visible spectrophotometry (wavelength: 360 nm)

### <Measurement Conditions of HPLC of Reference Examples 1 to 3 and Comparative Example 1>

Apparatus: Prominence (manufactured by SHIMADZU CORPORATION)
Column: Inertsil ODS (manufactured by GL Sciences Inc.)
Developing solvent: liquid A = acetonitrile, liquid B = 10 mM ammonium acetate aqueous solution (gradient elution: 0 min = liquid A 5%, 10 min = liquid A 50%)
Measurement sample: 1 mL of liquid I and 1 mL of developing solvent were mixed
Quantification method: performed by ultraviolet-visible spectrophotometry (wavelength: 254 nm)

### (Evaluation of Enteric Properties in Small Intestine)

A second solution for dissolution test (hereinafter, also referred to as "liquid II") was prepared by adding 1 volume of water to 1 volume of a phosphate buffer having a pH of 6.8. The pH of the liquid II was 6.8, which was equivalent to the pH in the small intestine.

The dissolution rate (%) (dissolution rate of the liquid II) of the medicinal ingredient eluted from the pharmaceutical composition into the liquid II was calculated by the same method as in the evaluation of gastric solubility.

### (Evaluation of Enteric Properties in Large Intestine)

A phosphate buffer (hereinafter, also referred to as "phosphate buffer solution") was prepared. The pH of the phosphate buffer solution was 7.4, which was equivalent to the pH in the large intestine.

The dissolution rate (%) (dissolution rate of the phosphate buffer solution) of the medicinal ingredient eluted from the pharmaceutical composition into the phosphate buffer solution was calculated by the same method as in the evaluation of gastric solubility.

The obtained results are shown in Table 11 below.

**[Table 11]**

| | Dissolution rate of liquid I | | | | Dissolution rate of liquid II | | | | Dissolution rate of phosphate buffer solution | | | |
|---|---|---|---|---|---|---|---|---|---|---|---|---|
| | 15 min | 30 min | 60 min | 120 min | 15 min | 30 min | 60 min | 120 min | 6 h | 24 h | 48 h | 72 h |
| Example 1 | 0 | 0 | 0 | 0 | 66.3 | 76.4 | 83.9 | 86.0 | - | - | - | |
| Example 2 | 0 | 0 | 0 | 0 | 40 | 49.3 | 55.5 | 61.4 | - | - | - | - |
| Example 3 | 0 | 0 | 0 | 0 | 67.6 | 87.1 | 98.5 | 97.9 | - | - | - | - |
| Example 4 | 0 | 3.9 | 7.7 | 12.4 | 0 | 3.5 | 9.1 | 13.5 | 29.8 | 45.5 | 50.3 | 52.1 |
| Example 5 | 2.8 | 2.8 | 2.9 | 3.3 | 32.6 | 40.9 | 49.9 | 58.9 | - | - | - | - |
| Example 6 | 5.1 | 9.2 | 11.1 | 8.8 | 0 | 0 | 0 | 0 | 51.7 | 69.9 | 77.9 | 80.3 |
| Example 7 | 4.6 | 15.6 | 17.5 | 18.7 | 2.6 | 4.2 | 12.3 | 19.7 | 46.9 | 70.8 | 80.1 | 80.1 |
| Example 8 | 2.7 | 13.1 | 16.3 | 18.5 | 1.4 | 3 | 4.2 | 8.3 | 27.7 | 49.4 | 59.9 | 59.9 |
| Reference Example 1 | 0 | 0 | 0 | 0 | 3.4 | 7.7 | 11.2 | 13.8 | 40.0 | 48.8 | 51.8 | 56.2 |
| Reference Example 2 | 0.2 | 0.4 | 0.5 | 0.8 | 5.8 | 8.8 | 12.3 | 16.7 | 36.0 | 46.0 | 49.1 | 52.2 |
| Reference Example 3 | 0.2 | 1.0 | 2.3 | 2.8 | 4.4 | 7.4 | 13.1 | 19.9 | 6.0 | 86.9 | 94.3 | 101.4 |
| Comparative Example 1 | 77.4 | 82.9 | 85.8 | 87.1 | 87.4 | 89.2 | 91.5 | 94.1 | - | - | - | - |

| | | | | | | | | | | | | |
|---|---|---|---|---|---|---|---|---|---|---|---|---|
| (unit: %) | | | | | | | | | | | | |

From the results in Table 11, it was found that Examples 1 to 8 show enteric properties. In Examples 1, 3, and 5 in which chitosan was not crosslinked, for example, the dissolution rate of the liquid I after 60 minutes was 20% or less, while the dissolution rate of the liquid II was 40% or more, and thus it was found that there was a tendency that chitosan was difficult to elute in the stomach and easy to elute in the small intestine. In Examples 4 and 6 to 8 in which chitosan was crosslinked, for example, the dissolution rate of the liquid I after 60 minutes was 20% or less, while the dissolution rate of the liquid II was 20% or less, and the dissolution rate of the phosphate buffer solution after 48 hours was 40% or more, and thus it was found that there was a tendency that chitosan was difficult to elute in the stomach and the small intestine and easy to elute in the large intestine.

In Reference Examples 1 to 3, it was found that the same effects as those of Examples 4, 6, 7, and 8 can be obtained regardless of the active ingredient of the pharmaceutical composition.

On the other hand, in Comparative Example 1, it was found that the dissolution rate of the liquid I was high and elution occurred in the stomach.

## Claims

1. A pharmaceutical composition comprising:
a core particle containing a medicinal ingredient and a dietary fiber; and
a coating particle layer coating the core particle.

2. The pharmaceutical composition according to claim 1, wherein the dietary fiber contains at least one selected from the group consisting of chitosan, chitin, cellulose, hemicellulose, lignin, and pullulan.

3. The pharmaceutical composition according to claim 1 or 2, wherein the core particle further contains a thickener.

4. The pharmaceutical composition according to claim 3, wherein the thickener contains at least one selected from the group consisting of inulin, pectin, a pectin processed product, guar gum, a guar gum decomposition product, psyllium seed gum, sterculia urens gum, tragacanth gum, gum arabic, indigestible starch, indigestible dextrin, isomaltodextrin, polydextrose, methyl cellulose, soybean polysaccharides, β-glucan, glucomannan, galactomannan, chondroitin sulfate, hyaluronic acid, levan, lignin, alginic acid and a salt thereof, agarose, gelatin, carrageenan, agar, hydroxypropylmethyl cellulose, hydroxypropylcellulose, an aminoalkyl methacrylate copolymer, and an ammonioalkyl methacrylate copolymer.

5. The pharmaceutical composition according to any one of claims 1 to 4, wherein a content of the dietary fiber is 1.0 mass% or more with respect to the total mass of the pharmaceutical composition.

6. The pharmaceutical composition according to any one of claims 1 to 5, wherein the coating particle layer contains a divalent or higher valent metal salt.

7. The pharmaceutical composition according to any one of claims 1 to 6, which is enteric.

8. The pharmaceutical composition according to any one of claims 1 to 7, wherein the dietary fiber is crosslinked.

9. The pharmaceutical composition according to claims 1 to 8, which is enteric in the large intestine.

10. The pharmaceutical composition according to any one of claims 1 to 9, wherein the medicinal ingredient contains at least one selected from the group consisting of mesalazine, salazosulfapyridine, prednisolone, azathioprine, tacrolimus, budesonide, 5-aminosalicylic acid, ustekinumab, sulfasalazine, an anti-TNF antibody, betamethasone, betamethasone sodium phosphate, betamethasone phosphate, azathioprine, 6-mercaptopurine, cyclosporine, and natalizumab.

11. A method for producing the pharmaceutical composition according to any one of claims 1 to 10, wherein the method comprising a granulation step of intermittently discharging a predetermined amount of an aqueous solution for granulation containing a medicinal ingredient, a dietary fiber, and water; charging droplets of the aqueous solution for granulation into a fluidized bed including a coating agent; and causing the coating agent to adhere to a surface of the droplets to allow moisture to be absorbed by the coating agent.

12. The method according to claim 11, further comprising a crosslinking step of crosslinking the dietary fiber with a crosslinking agent.
